# EUROPEAN PATENT APPLICATION

(11) **EP 4 825 437 A1**
(43) Date of publication of application: **21.10.2026**
(21) Application number: 25170748.5
(22) Date of filing: 15.04.2025
(51) Int. Cl.: A61N 5/10, G16H 20/40

(54) **A METHOD FOR INTERACTIVELY IMPROVING A RADIATION TREATMENT PLAN**

(71) Applicant: RaySearch Laboratories AB, 104 30 Stockholm (SE)
(72) Inventor: ENGWALL, Erik, 129 44 Hägersten (SE); NIESSEN, Tom, 169 37 Solna (SE); BYMAN, Lovisa, 171 69 Solna (SE)

(57) **Abstract**

The present disclosure relates to a method for improving an existing radiation treatment plan that uses a plurality of dose deposition points (2), for treating a target volume (4) in a patient, the method comprising:
- Providing (S01) a visual representation (10) of the target volume (4), exposed tissue or organs (6) and dose deposition points (2) in at least two dimensions, the visual representation (10) indicating a radiation dose distribution (8, 8', 8", 8‴, 8ʺʺ) of the existing radiation treatment plan;
- Enabling (S02) a cursor (18) on the visual representation (10), the cursor (18) being designed to choose a number of dose deposition points (2) via a virtual three-dimensional volume (14), whereby the chosen number of dose deposition points (2) are the ones falling within the virtual three-dimensional volume (14);
- modifying (S03) a treatment time and thus a dose, for each of the chosen number of dose deposition points (2) within the virtual three-dimensional volume (14) by either raising or lowering an input via the cursor (18);
- illustrating (S04) the change in radiation dose distribution (8', 8‴, 8ʺʺ) in real time in the visual representation (10) while the treatment time is modified.

## Description

### Technical field

The present disclosure pertains to the field of radiation therapy and, more particularly, to methods of refining an existing radiation treatment plan that employs a plurality of dose deposition points. It focuses on providing a visual interface to select, modify, and instantly display changes to dose deposition points treatment times (and thus the dose) for improved dose distribution in real time.

### Technical background

Radiation therapy for cancer treatment often involves delivering ionizing radiation through a series of discrete dose deposition points, such as "spots" in proton and light ion therapy or "dwell points" in brachytherapy, to precisely target a tumour while sparing surrounding organs. In external beam approaches such as pencil beam scanning, a particle or photon beam is aimed at specific locations (spots) in the target volume and the target volume is three-dimensionally painted with the spots. In brachytherapy, small radioactive seeds are inserted at different positions, which are called dwell points, in order to provide a dose that conforms to the target volume while sparing healthy tissue. Each spot or dwell point has a defined duration or intensity contributing to the total dose delivered.

Planning of these treatments relies heavily on tools that generate a pattern of dose deposition points with a defined duration, and thus dose levels, contained in an initial treatment plan. Dose distributions are commonly presented as two-dimensional slices through the anatomy, aiding in evaluating dose coverage and identifying critical structures. The slices can be provided in all three dimensions to give the clinician a comprehensive image of the target coverage and exposed healthy tissue.. However, adjusting these parameters to improve dose conformity or protect healthy tissue remains challenging even if an initial treatment plan is computed by the software. Current workflows often require re-optimizing large portions of the plan, which can be computationally intensive and may not provide immediate insight for a clinician into how localized changes affect the overall dose distribution.

Treatment teams may wish to selectively increase or decrease dose in certain regions choosing only specific dose deposition points. In this process, clinicians may rely on trial-and-error or repeated calculations to determine the impact of modifications - a process that can prolong planning cycles and limit fine-grained control over radiation dose sculpting. Existing systems generally lack the capability to make such local refinements while having control over which spots or dose deposition points are affected by real-time visualization of the dose deposition point selection. As a result, there is a need for more direct, efficient, and user-friendly methods to update existing treatment plans based on dose deposition points with rapid feedback on how those changes affect both target volume (tumour) coverage, healthy tissue exposure and dwell times.

### Summary of this disclosure

It is an object of this disclosure to provide an improved method for modifying existing radiation treatment plans, particularly for brachytherapy and/or pencil beam scanning, that allows for more intuitive and efficient adjustments to dose distributions within dose distribution points, while maintaining treatment efficacy and patient safety.

The method for improving an existing radiation treatment plan that uses a plurality of dose deposition points, for treating a target volume in a patient, comprises several steps. Initially, a visual representation of the target volume, exposed tissue or organs and dose deposition points is provided in at least two dimensions. This visual representation indicates a radiation dose distribution of the existing radiation treatment plan, allowing clinicians to see the effect of the existing radiation treatment plan. A cursor is enabled on the visual representation, the cursor being designed to choose a number of dose deposition points via a virtual three-dimensional volume. The virtual three-dimensional volume may thereby be hidden or visible on the visual representation. The chosen number of dose deposition points are the ones falling within this virtual three-dimensional volume.

The virtual three-dimensional volume may be hidden on the visual representation, or it may be visible for the clinician or user, especially if the virtual three-dimensional volume is adjusted manually.

An aspect of the method is the ability to modify a treatment time, and thus a dose, for each of the chosen number of dose deposition points within the virtual three-dimensional volume. This modification is achieved by either raising or lowering an input via the cursor or any other suitable input device. This allows for intuitive adjustment of treatment parameters, in particular treatment time.

The method illustrates, preferably interactively, the change in radiation dose distribution in real time in the visual representation while the treatment time is modified.

In an embodiment the size of the virtual three-dimensional volume may be chosen so that it always is possible to choose dose deposition points in at least two slices or slice images produced by the imaging modality of choice, e.g., computed tomography or magnetic resonance imaging, through at least a part of the target volume and organs at risk.

Real-time feedback allows clinicians or users to immediately see the effects of their adjustments, facilitating more informed decision-making on treatment plan improvements or changes.

In an embodiment of the method, the treatment times are changed depending on how close a dose deposition point of the chosen number of dose deposition points is arranged to a center of gravity of the virtual three-dimensional volume. The treatment time differences or deltas may increase towards the center of gravity of the virtual three-dimensional volume.

It is to be noted that the treatment time differences or deltas may be increased or decreased linearly instead of step-wise.

This feature allows for more nuanced adjustments to the dose distribution.

The existing radiation treatment plan may be a brachytherapy plan, wherein the dose deposition points are dwell points and wherein treatment times are dwell times.

The method may be particularly useful in brachytherapy planning, where precise control over dwell times is advantageous for treatment efficacy and improvements of radiation therapy plans.

The virtual three-dimensional volume used in the method may be a sphere or an ellipsoid.

These shapes provide intuitive and flexible selection volumes that can be easily manipulated in three-dimensional space. However, other shapes such as cuboids, cubes, cylinders or other geometric shapes may be used.

In some implementations, the virtual three-dimensional volume is displayed on the visual representation when the tool is active.

This visual feedback helps clinicians understand which dose deposition points or regions on the visual representation they are selecting and adjusting.

The cursor may be replaced by the virtual three-dimensional volume in some embodiments.

This can provide a more direct and intuitive interface for selecting and adjusting dose deposition points.

The virtual three-dimensional volume may be manually defined by user input.

This allows clinicians to have precise control over the selection volume based on their expertise and the specific requirements of each treatment plan.

The user input may be a distance between a periphery of the virtual three-dimensional volume and a point of the cursor or centre of the visual three-dimensional volume.

Another possible user input may be a specific number of dose deposition points, for example a lower limit number, that need to be selected for each real-time modification.

Alternatively, the virtual three-dimensional volume may be automatically determined by an algorithm based on the relative cursor position and positions of the dose deposition points, for example dose deposition points within a specific distance.

This can streamline the selection process and potentially identify optimal selection volumes based on the existing dose distribution and positions of dose deposition points.

In some implementations, the method includes the step of automatically adjusting a size of the virtual three-dimensional volume, so that it always encompasses a number of dose deposition point(s).

The number of dose deposition points may be based on a user input and may be a lower limit or an upper limit.

This feature ensures that the selected size of the virtual three-dimensional volume is always relevant and reasonable, regardless of the density of dose deposition points in different areas of the plan.

The method may allow for the treatment time for each dose deposition point of the chosen number of dose deposition points to increase by dragging the cursor in a first direction or decrease by sliding the graphical cursor in a second direction once the number of chosen dose deposition points are selected. This is typically a user input. It may however also be computed automatically depending on parameters specified by a clinician.

This provides an intuitive interface for fine-tuning the treatment times.

In a refinement of this feature, the treatment times may increase if the cursor is dragged along a negative local dose gradient and decrease if it is slid along the positive local dose gradient.

This allows for adjustments that naturally follow the existing dose distribution, potentially leading to more coherent and effective modifications.

The number of chosen dose deposition points via the three-dimensional volume may be illustrated in the visual representation as a number.

This provides clear feedback to the user or clinician about how many dose deposition points they are currently modifying.

The disclosure also extends to a graphical user interface designed to be used by clinicians or users. This graphical user interface comprises several elements that correspond to the method described above. It includes a visual representation of the target volume, exposed tissue or organs and dose deposition points in at least two dimensions, with the visual representation indicating a radiation dose distribution of the existing radiation treatment plan.

The graphical user interface also includes a cursor on the visual representation, designed to choose a number of dose deposition points via a virtual three-dimensional volume. It provides an option to modify a treatment time and thus a dose, for each of the chosen number of dose deposition points within the virtual three-dimensional volume by either raising or lowering an input via the cursor. Finally, it offers an illustration of the change in radiation dose distribution in real time in the visual representation while the treatment time is modified.

The disclosure further includes a computer program product comprising computer readable means which, when executed in a computer, will cause the computer to perform the method described above. This allows for the method to be implemented in existing radiation therapy planning systems.

Lastly, the disclosure describes a radiation therapy planning system comprising a processor, a memory, wherein the memory comprises the computer program product mentioned above. This system is designed to perform the method for improving existing radiation treatment plans.

This method and associated systems provide several benefits. They allow for more intuitive and efficient adjustments to radiation treatment plans, particularly in brachytherapy. The use of a virtual three-dimensional volume for selecting multiple dose deposition points simultaneously can save time and reduce the potential for errors compared to individual point selection. The real-time visualization of changes to the dose distribution allows for immediate feedback, potentially leading to better-optimized treatment plans. The ability to adjust treatment times based on the position within the selection volume and along dose gradients allows for nuanced modifications that can better conform to the desired dose distribution. Overall, this method has the potential to improve the efficiency and effectiveness of radiation treatment planning, ultimately benefiting patient outcomes.

In an embodiment the visual representation may comprise three orthogonal views of the target volume and surrounding tissues to cover all three dimensions. The dose change in real time may also be illustrated on all three orthogonal views.

In this disclosure, certain terms and technical specifications will be used. Some of them are explained referring to the figures and some of them are herewith explained and specified for optimal understanding of the described disclosure:

### Visual Representation

The term "visual representation" refers to a graphical depiction of the target volume, exposed tissue, organs, skeleton and dose deposition points in at least one dimension on at least two slices through the target volume and patient. This representation indicates the radiation dose distribution of the existing or initial radiation treatment plan. The visual representation encompasses any form of graphical display that can visually convey the distribution of radiation doses across the target volume and surrounding areas. The visual representation specifically refers to the images or graphics displayed on a screen or monitor that allow a clinician/user to view and interact with the radiation treatment plan. The visual representation may comprise a graphical user interface or it may be a graphical user interface.

### Cursor

A " cursor" is an interactive tool displayed on the visual representation that allows the user to select a number of dose deposition points within the visual representation. The cursor may be a graphical cursor, a pointer or indicator used to interact with a graphical user interface and/or the visual representation. The term cursor may refer to a specific tool designed to choose dose deposition points within a virtual three-dimensional volume on the visual representation, enabling the clinician or user to modify treatment parameters for those dose deposition points. The cursor may be called virtual cursor. The cursor may be steered via a mouse, a joystick or a trackpad or any other suitable device.

### Dose deposition points

In the context of radiation therapy, "dose deposition points" refer to specific locations within the target volume or within the patient, where radiation or radiation dose is delivered. The term dose deposition points can refer to any designated point or area within a treatment plan where radiation is applied and delivered. This also includes the fact that the radiation dose is typically extending as a sphere or a volume from and around the actual dose deposition point. The dose deposition point therewith describes the point where radiation is deposited, either via pencil beam scanning or Brachytherapy. A dose deposition point may be originating from a radiation source that is stopped for certain period in time, such as in Brachytherapy, where a dose deposition point is a dwell point, or from pencil beam scanning, where the dose deposition points are spots that paint a target volume using a pencil beam. The term dose deposition point refers to the precise locations within the target volume that are selected for modification of treatment time and, consequently, dose. Again, the dose will be deposited centered around the precise location or position of the dose deposition point, thereby gradually decreasing radially from the point. Treatment time or intensity of the dose deposition points may be modified, and this affects the three-dimensional distribution of the radiation dose. The dose deposition points may be called spots. In case of Brachytherapy, the dose deposition points are the dwell points, where a source consisting of radioactive material is temporarily stopped for a certain time period, which is the treatment time. During this treatment time the source of radioactive material releases its dose.

### Treatment Time or dwell time

"Treatment time" or "dwell time" refers to the duration for which radiation is applied to each of the chosen dose distribution points within the virtual three-dimensional volume. In a broad sense, it encompasses any time-related parameter that affects the delivery of radiation. It may specifically refer to the adjustable parameter relating to a difference or delta in treatment time that can be increased or decreased via the cursor or another input method to modify the dose delivered to the chosen dose deposition points.

Deltas or differences may be continuously increased or decreased at each dose distribution points.

### Dose

The term "dose" refers to the amount of radiation delivered to a specific dose deposition point or volume within the patient. Broadly, it encompasses any measure of radiation exposure within the treatment plan. In a narrower sense, it refers to the specific quantity of radiation that is adjusted by modifying the treatment time for the chosen dose deposition point within the virtual three-dimensional volume. As mentioned previously, the dose or radiation dose is typically extending three-dimensionally around a dose deposition point.

### Virtual Three-Dimensional Volume

A "virtual three-dimensional volume" is a conceptual space within the visual representation that encompasses a chosen number of dose deposition points. Broadly, it can refer to any three-dimensional volume used for selecting and modifying treatment parameters. It may refer a specific geometric shape, such as a sphere or ellipsoid or another three-dimensional shape, used to define the volume within which dose distribution points are selected for dose modification. The virtual three-dimensional volume may be visible or hidden on the visual representation or graphical user interface. Generally, the virtual three-dimensional volume may be dynamically adjusted. Typically, the virtual three-dimensional volume may be indicated by showing the edges or by illustrating it with a lighter colour or the like. The target volume is in some cases depicted on several slices, originating from a computer tomography or MRI for example, the three-dimensional volume may thereby include dose deposition points located in at least two such slices generated by any suitable imaging modality such computed tomography or magnetic resonance imaging. The at least two slices may be neighbouring or adjacent slices.

### Interactive Illustration

"Interactive illustration" refers to the real-time visual feedback provided on the visual representation as the treatment time is modified. It may encompass any dynamic display that updates in response to user input. It may specifically refer to the immediate changes shown in the radiation dose distribution on the visual representation as the clinician adjusts the treatment time for the chosen dose deposition points.

### Dwell Points

In the context of brachytherapy, "dwell points" refer to specific locations where a radiation source is temporarily placed to deliver a dose, and "dwell times" refer to the duration for which the source remains at each point, please see also definition of "treatment time". These terms can refer to any point and time-related parameters in a brachytherapy plan.

### Negative local dose gradient

The negative local dose gradient describes how treatment times may increase if the cursor is slid along a gradient that comes from longer treatment times and therewith higher dose towards a region or gradient with shorter treatment times and therewith a lower dose. Thus, from a clinician or user's point of view, the clinician is choosing a region with a certain radiation dose (treatment time(s)) and extends that region towards the area or region where the cursor is drawn or slid to. In other words, if an isodose line is dragged over the target area or target volume, then that specific isodose is extended.

### Positive local dose gradient

The positive local dose gradient describes how treatment times may decrease if the cursor is slid along a gradient that comes from shorter treatment times and therewith lower dose towards a region or gradient with longer treatment times and therewith a higher dose. Thus, from a clinician or user's point of view, the clinician is choosing a region with a certain radiation dose (treatment time(s)) and extends that region towards the area or region where the cursor is drawn or slid to. In other words, if an isodose line is dragged over the target area or target volume, then that specific isodose is extended.

The positive and negative dose gradients may enable an intuitive adaption of the dose gradients on the visual representation.

The disclosure will now be further explained referring to the figures.

### Brief Description of the Drawings

The disclosure will be described in more detail in the following, by way of example and with reference to the appended drawings, in which
- Figure 1: schematically illustrates a method according to this disclosure;
- Figure 2: schematically illustrates a radiation therapy treatment planning system according to this disclosure;
- Figure 3: schematically illustrates a single dose deposition point and a target volume;
- Figure 4: schematically illustrates a similar as figure 2 but with another, increased, treatment time in the dose deposition point;
- Figure 5: schematically illustrates dose deposition points and multiple isodose lines;
- Figure 6: schematically illustrates an increased treatment time and therewith dose distribution of the dose deposition points of figure 3;
- Figure 7: schematically illustrates a treatment time distribution over various dose deposition points;
- Figure 8: schematically illustrates a similar view as figure 5 but with a different treatment time distribution;
- Figure 9: schematically illustrates a visual representation of a target volume and organs at risks and a dose distribution prior to modification;
- Figure 10: schematically illustrates a similar view as figure 7 but with a virtual three-dimensional volume visible;
- Figure 11: schematically illustrates a similar view as figure 8, where a certain number of dose deposition points are selected for treatment time modification;
- Figure 12: schematically illustrates a similar view as figure 9 and how treatment times of the selected dose depositions points may be decreased;
- Figure 13: schematically illustrates a visual representation of the target volume and organs at risk and the radiation dose distribution after the decreased treatment times have been applied to the selected dose deposition points;
- Figure 14: schematically illustrates a similar view as figure 9 but this time the three dimensional volume is placed in another area and different dose deposition points are selected;
- Figure 15: schematically illustrates a similar view as figure 12 and how treatment times of the selected dose depositions points may be increased;
- Figure 16: schematically illustrates a visual representation of the target volume and organs at risk and the radiation dose distribution after the increased treatment times have been applied to the selected dose deposition points;
- Figures 17a and 17b: schematically illustrate how the cursor can be slid along a positive dose gradient to modify the radiation dose distribution; and
- Figures 18a and 18b: schematically illustrate how the cursor can be slid along a negative dose gradient to modify the radiation dose distribution.

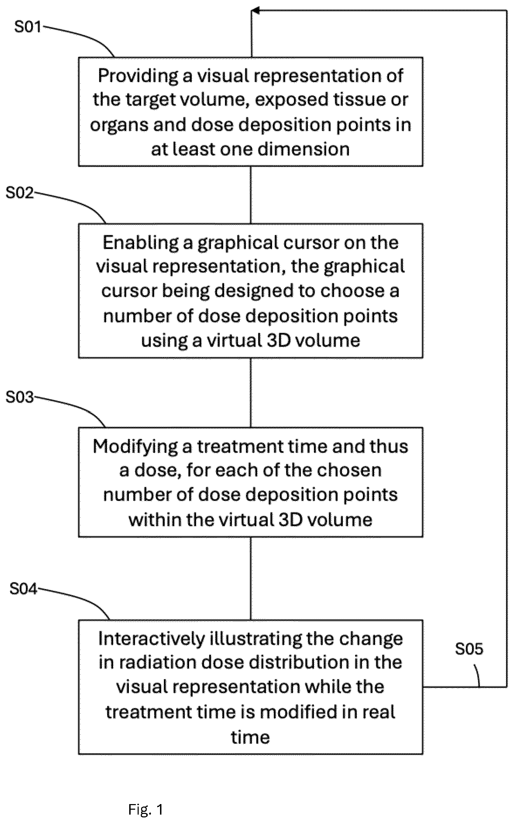

Figure 1 illustrates a method for improving an existing radiation treatment plan that uses a plurality of dose deposition points, for treating a target volume in a patient, the method comprising:
- providing S01 a visual representation of the target volume, exposed tissue or organs and dose deposition points in at least two dimensions the visual representation indicating a radiation dose distribution of the existing radiation treatment plan;
- enabling S02 a cursor on the visual representation, the cursor being designed to choose a number of dose deposition points via a virtual three-dimensional volume, whereby the chosen number of dose deposition points are the ones falling within the virtual three-dimensional volume;
- modifying S03 a treatment time and thus a dose, for each of the chosen number of dose deposition points within the virtual three-dimensional volume by either raising or lowering an input via the cursor;
- illustrating S04 the change in radiation dose distribution in real time in the visual representation while the treatment time is modified.

The illustrating step S04 may be interactive and show the change of radiation dose distribution in real time. Further, the changes to the radiation treatment plan may be saved and then the steps S01 to S04 may be repeated S05.

The step of changing or modifying S03 the treatment times may take into account on how close a dose deposition point of the chosen number of dose deposition points is arranged to a center of gravity of the virtual three-dimensional volume and wherein the treatment time differences that are applied increase towards the center of gravity of the virtual three-dimensional volume. This is also illustrated and explained referring to 8.

The existing radiation treatment plan may be a brachytherapy plan and the dose deposition points may be dwell points and treatment times may be dwell times.

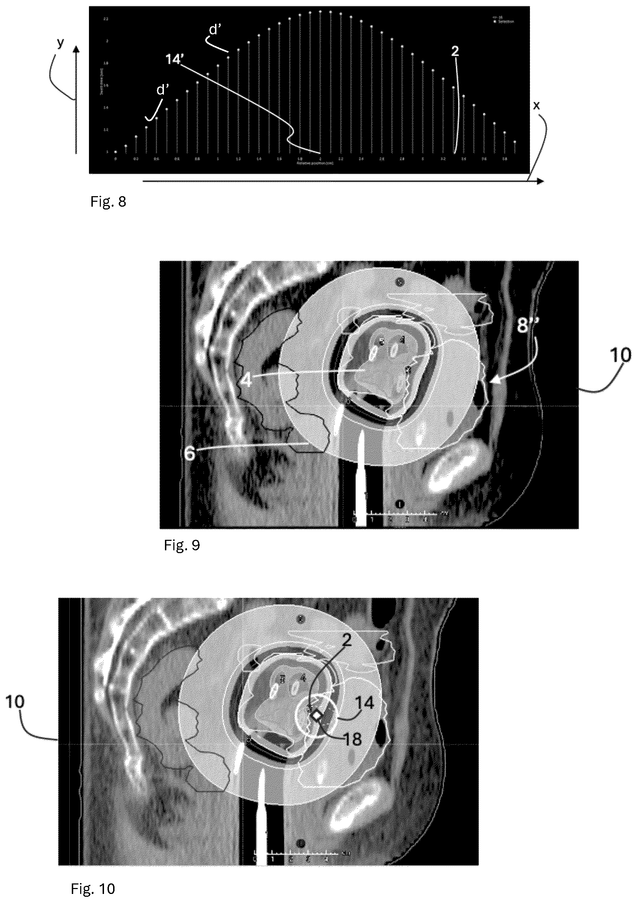

The virtual three-dimensional volume may be a sphere, as explained referring to figure 10, or an ellipsoid.

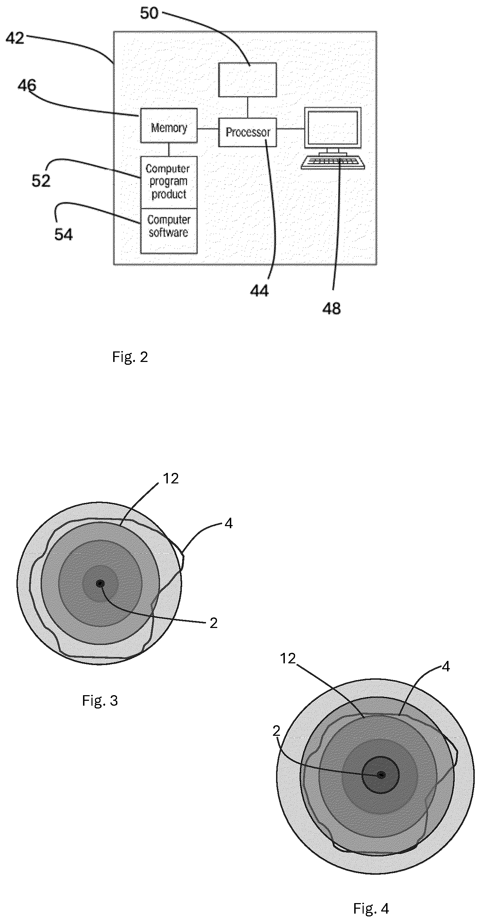

Figure 2 illustrates further a radiation therapy treatment planning system 42 comprising a processor 44, a memory 46 connected to the processor 44, an optional extra memory 50 also connected to the processor 44 and/or the memory 46 and an input/output interface 48 having a display and/or a keyboard for user interaction. The processor 44 may be any combination of a central processing unit (CPU), multiprocessor, digital signal processor (DSP), an ASIC (application specific integrated circuit) capable of executing software instructions stored on the memory and capable of executing the methods described according to the embodiments herein. The memory 46 and/or the extra memory 50 may comprise software code in the form of a computer program product 52 comprising computer software 54 or instructions that comprise any method according to this disclosure and that can perform any method according to this disclosure. The computer software 54 may be executed by the processor 44.

The memory 46 and the extra memory 50 may be any of a random access memory (RAM) or read only memory (ROM) or any combination thereof.

The input/output interface 48 may be a graphical user interface comprising a visual representation 10 (as shown from figure 2 onwards). The graphical user interface may be a touch screen.

Referring to figures 3 and 4 the concept disclosed herein is now explained and becomes clearer to understand. Figures 3 and 4 both illustrate a target volume 4 and a single dose deposition point 2 in which a radiation source is placed for a treatment in order to provide the dose distribution illustrated in figure 3. Figure 3 illustrates isodose lines 12 positioned concentric around the dose deposition point 2 depending on a chosen first treatment time. If the chosen treatment time is increased, then the isodose lines 12 change and the radiation dose around the dose deposition point 2 increases. As one can understand from observing figures 3 and 4, the radiation dose and its range increase for each dose distribution point 2 if the treatment time or dwell time is increased. Likewise, the radiation dose and its range are decreased if the treatment is decreased. Illustrating dose distributions based on such changes in treatment times or dwell times in chosen dose deposition points, is what this disclosure is about.

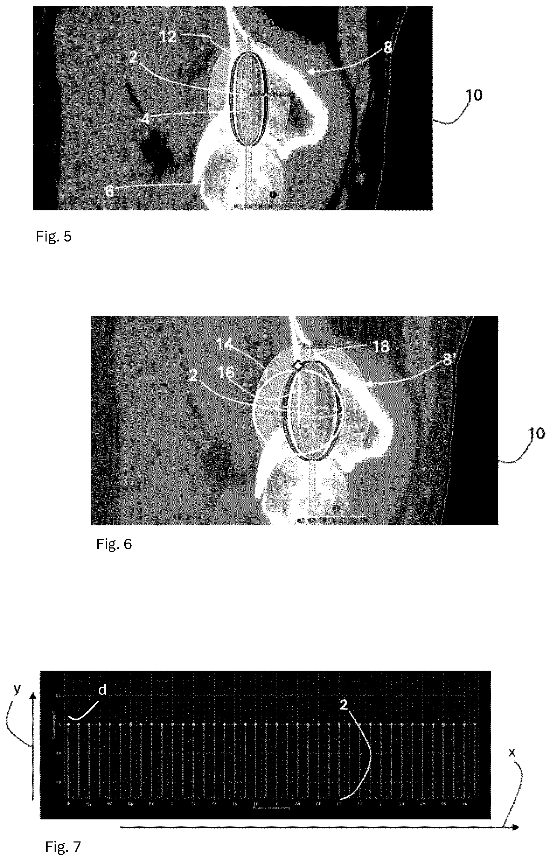

Figure 5 illustrates a visual representation 10 of a target volume 4, organs at risk 6, dose deposition points 4 arranged within a single needle or catheter having a certain treatment time and a corresponding radiation dose distribution 8 including isodose lines 12 that illustrate the different levels of radiation dose across the target volume 4, the organs at risk 6 and surrounding tissue. A radiation source may be moved along the needle and stopped for selected times at various dose deposition points in order to distribute the radiation dose. The illustrated case concerns dwell points with a dwells time for brachytherapy. Once the dose deposition points 2 along the needle or catheter are selected and their treatment times , for example dwell time, are increased then the radiation dose distribution 8 changes to a new radiation dose distribution 8' as shown in figure 6, which delivers a higher dose due to increased treatment times in the single dose deposition points 2.

Figure 6 further illustrates a virtual three-dimensional volume 14 that encompasses and selects dose distribution points 2 distributed along a needle similar to figure 5. A straight line or adjustment indicator 16 that schematically illustrates an upwards drag of a cursor 18 in order to increase a treatment time or treatment change/delta in the selected dose deposition points 2, is also shown in figure 6. The upwards dragging - increase may also be the other way round, thus lead to a decrease in treatment time change or delta. As can be seen, the increase in treatment time delta or increase in treatment time leads to a new radiation dose distribution 8' that is directly visible for the clinician, in real time.

In figure 6, the virtual three-dimensional volume virtual three-dimensional volume 14 is displayed on the visual representation 10 when the tool is active. It may however also be hidden and only the cursor 18 visible during the selection process, as explained later herein. Alternatively, the cursor 18 may be completely replaced by the virtual three-dimensional volume 14 during the selection of dose deposition points.

Figure 7 illustrates dwell times along dwell points. The treatment times are shown on the y-axis and the respective position of the dose deposition points 2 and thus distance on the x-axis. As indicated in figure 7 the increase (or decrease) of the treatment time delta d may be linear across all selected dose deposition points. Linearly increasing the treatment time d in all dose deposition points 2 increase the dose across all areas similarly.

Figure 8 illustrates a different pattern of treatment time deltas. The y- and x-axis are also shown and refer again to treatment time on the y-axis and the position of the dose deposition points 2 on the x-axis. As can be seen from figure 8, the treatment time deltas d' may increase nonlinearly and chosen to be greater towards a center of gravity 14' of the virtual three-dimensional volume (not shown in 8) following a parabolical or gauss curve.

As explained referring to figures 7 and 8 the treatment time deltas may be adjusted depending on various patterns or geometric curves, of which figures 7 and 8 show two examples. Other curves or adjustment patterns of the treatment time deltas fall within the scope of this disclosure.

Figure 9 illustrates a visual representation 10 of the prostate, as target volume 4, with the radiation dose distribution 8" as illustrated based on the existing radiation treatment plan.

Organs at risk 6 are also visible in the visual representation 10 or graphical user interface. Now for the sake of explanation let's assume that the clinician wants to modify the dose in the radiation dose distribution of figure 9 by using the virtual three-dimensional volume 14 and the cursor 18 to select dose deposition points 2 for modification, as shown in figure 10. The virtual three-dimensional volume 14 is used to select a number of dose deposition points 2 via the cursor 18, for example by dragging the cursor by holding down a key or the mousepad in order to choose a size of the virtual three-dimensional volume 14 and there a number of dose deposition points 2 to be included. The virtual three-dimensional volume 14 may be manually defined by user input.

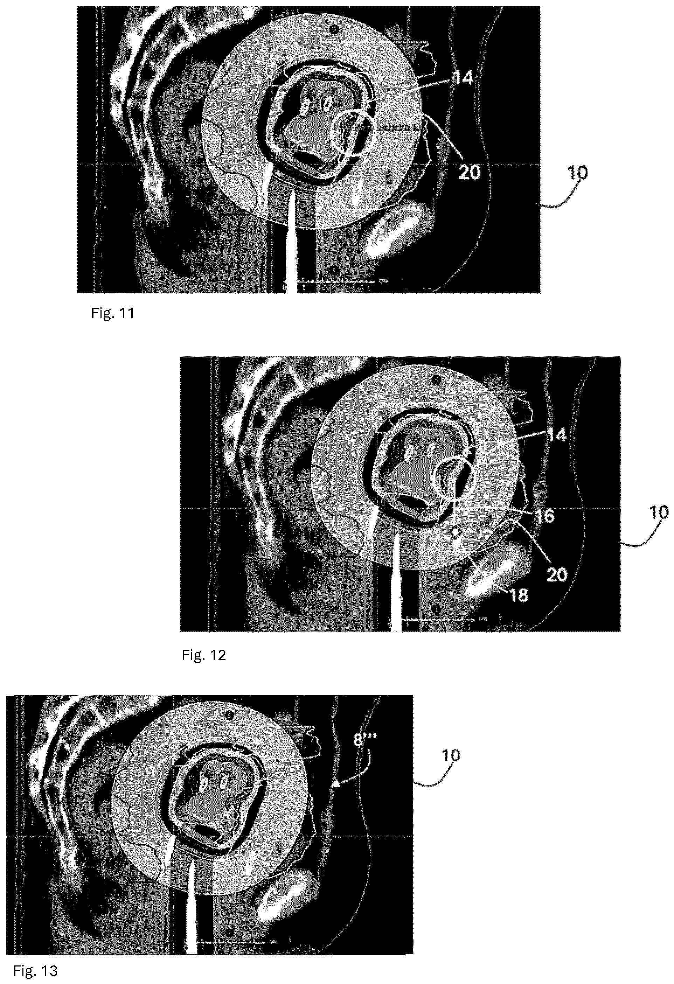

Once the clinician chose the size of the virtual three-dimensional volume 14, the number 20 of chosen dose deposition points is illustrated on the visual representation 10, as shown in figure 11.

In order to decrease the treatment time, the clinician then slides or drags the cursor 18 downwards along an adjustment indicator 16, in the shown example in figure 12. This sliding of the cursor 18 leads to a change in the radiation dose distribution 8‴ as illustrated in figure 13, which change in radiation dose distribution 8‴ is visible when comparing figure 13 with figure 9. Thus, it is very intuitive and straightforward for the clinician to improve or modify the existing radiation treatment plan and the changes in radiation dose distribution 8', 8‴ are shown in real time on the visual representation 10.

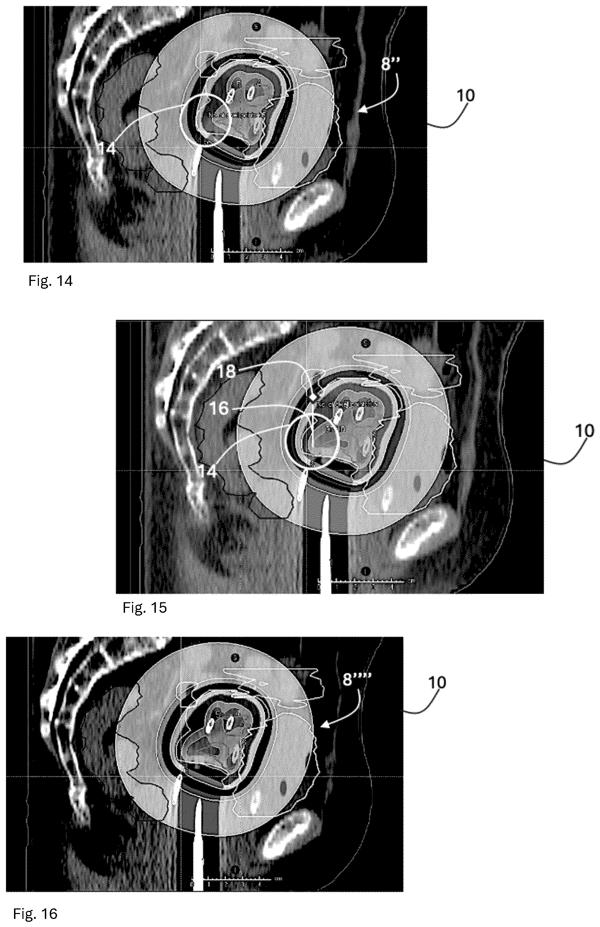

Turning now to figure 14, which illustrates a visual representation similar to figure 9, with the same dose distribution 8". The virtual three-dimensional volume 14 is also illustrated and the number of dose deposition points are chosen, the number is indicated in the virtual representation, as described referring to the previous figures. In this case, however and as shown in figure 15, the clinician wants to increase the dose in the selected number of dose deposition points and region, respectively, by dragging the cursor 18 upwards along the adjustment indicator 16.

This modification of sliding the cursor 18 upwards along the adjustment indicator 16, is leading a new radiation dose distribution 8"", as shown in figure 16. In this new radiation dose distribution 8"", the dose in the volume affected by the virtual three-dimensional volume 14 is increased, as shown and to be seen when comparing figures 14 and 16.

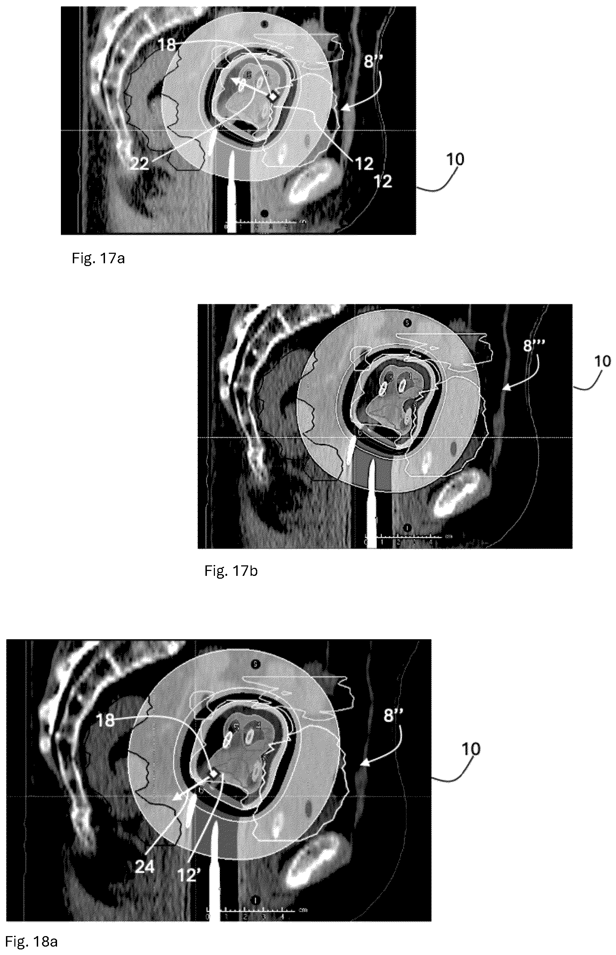

Referring now to figures 17a and 17b, an alternative solution of modifying the radiation dose distribution of an existing radiation therapy treatment plan is illustrated. The cursor 18 is placed in an area with a certain isodose line 12, once the clinician indicates, for example by a click, that this is the isodose line 12 that is selected, the cursor 18 may then be dragged along a positive dose gradient 22 to decrease the dose by dragging the chosen isodose line 12 along the positive dose gradient 22. Thereby the radiation dose along the positive dose gradient 22 is reduced, as illustrated by the existing radiation dose distribution 8" of figure 17a and the new dose distribution 8‴ of figure 17b. In the example the virtual three-dimensional volume (not shown) is hidden and may for example by calculated automatically by an algorithm or it the size may be chosen by the clinician. The algorithm may take the relative cursor position and positions of the dose deposition points into account to select an optimal number of dose deposition points for modification.

Figures 17a and 17b correspond to the example illustrated referring to figures 9 to 13, showing an alternative procedure.

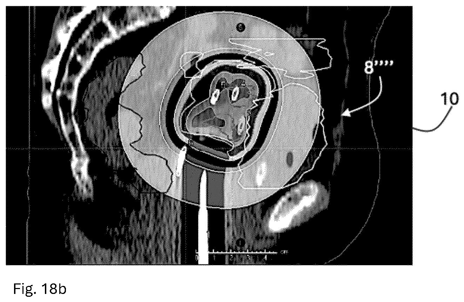

Figures 18a and 18b, show a similar solution as figures 15a and 15b but this time the cursor 18 is clicked or similar and then slid along a negative dose gradient 24 in order to "extend" the dose of the iso line 12' along the negative dose gradient 24, thereby increasing the dose in the selected region and dose deposition points, respectively. This leads to a new radiation dose distribution 8"" as shown in figure 18b.

Referring back to the figures, the size of the virtual three-dimensional volume 14 may by automatically adjusted so that it always encompasses a number of dose deposition point(s), whereby the number of dose deposition points is based on a user input. Also, the virtual three-dimensional volume 14 is illustrated as a sphere in the figures, any other suitable shape may however be chosen.

Further, the adjustment indicator 16, the positive dose gradient 22 and the negative dose gradient 24 may be referred to as first - and second directions, respectively.

From the embodiments illustrated in the figures it becomes clear that various combinations of cursors and methods as described fall within the scope of this disclosure. The methods described in the figures may be combined with one another and certain steps may be omitted.

## Claims

1. A method for improving an existing radiation treatment plan that uses a plurality of dose deposition points (2), for treating a target volume (4) in a patient, the method comprising:
- Providing (S01) a visual representation (10) of the target volume (4), exposed tissue or organs (6) and dose deposition points (2) in at least two dimensions, the visual representation (10) indicating a radiation dose distribution (8, 8', 8", 8"', 8"") of the existing radiation treatment plan;
- Enabling (S02) a cursor (18) on the visual representation (10), the cursor (18) being designed to choose a number of dose deposition points (2) via a virtual three-dimensional volume (14), whereby the chosen number of dose deposition points (2) are the ones falling within the virtual three-dimensional volume (14);
- modifying (S03) a treatment time and thus a dose, for each of the chosen number of dose deposition points (2) within the virtual three-dimensional volume (14) by either raising or lowering an input via the cursor (18);
- interactively illustrating (S04) the change in radiation dose distribution (8', 8‴, 8"") in real time in the visual representation (10) while the treatment time is modified.

2. The method according to claim 1, comprising the step of changing the treatment times depending on how close a dose deposition point (2) of the chosen number of dose deposition points (2) is arranged to a center of gravity (14') of the virtual three-dimensional volume (14) and wherein treatment time deltas that are applied increase towards the center of gravity (14') of the virtual three-dimensional volume (14).

3. The method according to any of the previous claims 1 or 2, wherein the existing radiation treatment plan is a brachytherapy plan, wherein the dose deposition points (2) are dwell points and wherein treatment times are dwell times.

4. The method according to any of the previous claims 1 to 3, wherein the virtual three-dimensional volume (14) is a sphere or an ellipsoid.

5. The method according to any of the previous claims 1 to 4, wherein the virtual three-dimensional volume (14) is displayed on the visual representation (10).

6. The method according to any of the previous claim, wherein the cursor (18) is replaced by the virtual three-dimensional volume (14).

7. The method according to any of the previous claims 1 to 6, wherein the virtual three-dimensional volume (14) is manually defined by user input.

8. The method according to any of the previous claims 1 to 7, wherein the virtual three-dimensional volume (14) is automatically determined by an algorithm based on the relative cursor (18) position and positions of the dose deposition points (2).

9. The method according to claim 8, comprising the step of automatically adjusting a size of the virtual three-dimensional volume (14), so that it always encompasses a number of dose deposition point(s) (2), whereby the number of dose deposition (2) points is based on a user input.

10. The method according to any of the previous claims 1 to 9, wherein the treatment time for each dose deposition point (2) of the chosen number of dose deposition points (2) increases by sliding the cursor in a first direction (16, 22) or decreases by sliding the graphical cursor in a second direction (16, 24) once the number of chosen dose deposition points (2) is selected.

11. The method according to claim 10, wherein the treatment times increase if the cursor (18) is slid along a negative local dose gradient (24) and decrease if it is slid along the positive local dose gradient (22).

12. The method according to any of the previous claims, wherein the number of chosen dose deposition points (2) via the three-dimensional volume (14) is illustrated in the visual representation (14) as a number (20).

13. A graphical user interface (48) designed to be used by clinicians, the graphical user interface comprising:
- a visual representation (10) of the target volume (4), exposed tissue or organs (6) and dose deposition points (2) in at least two dimensions, the visual representation (10) indicating a radiation dose distribution (8, 8', 8", 8‴, 8ʺʺ) of the existing radiation treatment plan;
- a cursor (18) on the visual representation (10), the cursor (18) being designed to choose a number of dose deposition points (2) via a virtual three-dimensional volume (14), whereby the chosen number of dose deposition points (2) are the ones falling within the virtual three-dimensional volume (14);
- an option to modify a treatment time and thus a dose, for each of the chosen number of dose deposition points (2) within the virtual three-dimensional volume (14) by either raising or lowering an input via the cursor (18);
- an illustration of the change in radiation dose distribution (8', 8‴, 8"") in real time in the visual representation (10) while the treatment time is modified.

14. A computer program product (52) comprising computer readable means (54) which, when executed in a computer, will cause the computer to perform the method according to any one of the preceding claims 1 to 12.

15. A radiation therapy planning system comprising a processor (44), a memory (46), wherein the memory (44) comprises a computer program product (52) according to the previous claim, the radiation therapy planning system is designed to perform the method according to any of the preceding claims 1 to 12.
